# EUROPEAN PATENT APPLICATION

(11) **EP 4 850 536 A1**
(43) Date of publication of application: **09.12.2026**
(21) Application number: 25751457.0
(22) Date of filing: 05.02.2025
(51) Int. Cl.: A23L 33/18, A61K 31/198, A61K 38/08, A61P 21/00

(54) **METHOD AND COMPOSITION FOR ENHANCING OR IMPROVING MUSCLE FUNCTION AND/OR IMPROVING ATHLETIC PERFORMANCE AND/OR PREVENTING OR ALLEVIATING FATIGUE**

(30) Priority: 06.02.2024 WO PCT/CN2024/076238; 06.02.2024 WO PCT/CN2024/076246; 21.08.2024 WO PCT/CN2024/113549
(71) Applicant: Nanjing Nutrabuilding Bio-Tech Co., Ltd., Nanjing, Jiangsu 210017 (CN)
(72) Inventor: YI, Ronghua, Nanjing, Jiangsu 210017 (CN); ZHANG, Qianjing, Nanjing, Jiangsu 210017 (CN); WELLS, Shawn, Nanjing, Jiangsu 210017 (CN); LIAO, Kylin, Nanjing, Jiangsu 210017 (CN)
(74) Representative: Lorenz Seidler Gossel Part. mbB
(86) International application number: PCT/CN2025/075662
(87) International publication number: WO 2025/167857

(57) **Abstract**

A method for enhancing or improving muscle function and/or improving athletic performance and/or preventing or alleviatingalleviating fatigue and/or increasing or improving a nitric oxide signal or level. The method comprises: administering to a subject acetyltaurine or a hydrate, salt, ester, solvate, or derivative thereof, or an arginine dipeptide or a salt, ester, or derivative thereof, or a mixture of the two.

## Description

### TECHNICAL FIELD

The present invention relates to the technical field of nutritional products or dietary supplements, and particularly relates to a method and a composition for enhancing or improving muscle function and/or improving exercise performance and/or preventing or alleviating fatigue and/or increasing or improving nitric oxide signal or level.

### BACKGROUND

As we all know, exercise has many benefits, such as improving quality of life, optimizing heart and blood vessel health, controlling blood pressure, optimizing brain health and memory, increasing happiness, strengthening bones and muscles, reducing joint stiffness, reducing the risk of falling, and optimizing sleep quality. In addition, exercise has a positive effect on muscle strength and overall muscle tension. A study concluded that exercise has a moderate to significant impact on the muscle strength, body composition, physiological function and inflammatory response of the elderly. Therefore, it is very important to enhance or improve exercise/athletic/sports performance and to improve muscle function.

In order to improve exercise performance, many people who regularly exercise and lift weights will choose to take supplements. However, the existing products have many side effects (such as palpitation, rapid heartbeat, etc.) and the effect is not ideal. Therefore, it is very important to find products that are efficient and have no side effects.

Nitric oxide (NO), also known as nitrogen oxides, is produced by almost every cell in the human body. It is considered as a medium of intercellular communication and plays an important role in many processes of the body, including inflammation, vasodilation and nerve transmission. First of all, when it comes to our vascular health, it is considered as one of the most important molecules. For example, NO plays a key role in vasodilation and can also lower blood pressure. NO is a signal molecule widely existing in cardiovascular system and various cell types, and it can prevent the occurrence and development of atherosclerosis in many ways. Simply put, NO can improve endothelial function. NO also has the function of scavenging oxygen free radicals, including preventing the oxidation of low-density lipoprotein cholesterol. NO can regulate the central nervous system and improve nerve degeneration. NO can also improve the tolerance to aerobic and anaerobic exercise. NO plays an important role in regulating glucose uptake in skeletal muscle during contraction/exercise. Therefore, it is particularly important to enhance the signal of NO.

At present, products that enhance NO signal in the market will produce side effects, such as nausea, stomach upset and palpitation, so it is very important to find a product that can enhance NO signal without side effects.

### SUMMARY

One aspect of the present invention relates to a method for enhancing or improving muscle function and/or improving exercise performance and/or preventing or alleviating fatigue and/or increasing or improving nitric oxide signal or level, where the method comprises: administering acetyltaurine or a hydrate, salt, ester, solvate, derivative thereof; or diarginine or a salt, ester, derivative thereof; or a mixture of both to a subject. In some embodiments, an effective amount of acetyltaurine or a hydrate, salt, ester, solvate, derivative thereof; or diarginine or a salt, ester, derivative thereof; or a mixture of both, is administered to the subject. In some embodiments, the enhancing or improving muscle function includes one or more of the following: alleviating or preventing muscle dysfunction, improving mitochondrial function in muscle, improving insulin resistance in muscle, increasing muscle mass, increasing muscle strength and endurance, improving glucose transport and metabolism in skeletal muscle, alleviating or preventing muscle injury, and reducing muscle fatigue; the improving exercise performance includes one or more of the following: increasing running distance, increasing exercise time, increasing running speed, improving exercise endurance or explosive force, reducing inflammation and muscle fatigue after exercise, and improving muscle contraction during exercise; the preventing or alleviating fatigue includes preventing physical fatigue after exercise and accelerating recovery after exercise.

In some embodiments, the method of the present invention can increase the level of nitrite and/or cyclic guanosine monophosphate.

In some embodiments, the method enhances or improves muscle function and/or improves exercise performance and/or prevents or alleviates fatigue by improving nitric oxide level or signal, or increasing the level of cyclic guanosine monophosphate (cGMP), or increasing the level of nitrite.

In some embodiments, compared with subject not receiving administration, the enhancement or improvement in muscle function or exercise performance and/or the prevention or alleviation of fatigue in subject of administration group is more than 10%, 30%, 40%, 50%, 60%, 70% or 90%.

In some embodiments, compared with exercise group not receiving administration, the exercise time of administration group increases more than 10%, 30%, 45%, 50%, 60%, 70% or 90%. Compared with exercise group not receiving administration, the running distance of administration group increases more than 10%, 20%, 30%, 35% or 50%. Compared with exercise group not receiving administration, the urea nitrogen level in administration group decreases more than 10%, 15%, 20%, 30% or 40%. Compared with exercise group not receiving administration, the liver glycogen level in administration group increases more than 10%, 15%, 20%, 30% or 40%.

In some embodiments, compared with control subject not receiving administration, nitric oxide signal or level in subject of administration group increases or improves more than 10%, 30%, 40%, 50%, 60%, 70%, 90%, 120%, 150% or 180%.

In some embodiments, the subject is human or mammal.

In some embodiments, the acetyltaurine or hydrate, salt, ester, solvate, derivative thereof; or the diarginine or salt, ester, derivative thereof; or the mixture of both may be formulated as nutritional product, dietary supplement, food or beverage.

In some embodiments, the acetyltaurine or hydrate, salt, ester, solvate, derivative thereof; or the diarginine or salt, ester, derivative thereof; or the mixture of both, is prepared into solid preparation or liquid preparation.

In some embodiments, the acetyltaurine or hydrate, salt, ester, solvate, derivative thereof; or the diarginine or salt, ester, derivative thereof; or the mixture of both, is in the form of lotion, paste, suppository, tablet, pill, granule, powder, film, capsule, beverage, aerosol, elixir, tincture, tonic, liquid suspension, syrup.

In some embodiments, the acetyltaurine or hydrate, salt, ester, solvate, derivative thereof is administered at a dosage of 0.1 µM to 1 M or 0.05-20 g or 0.001 to 90.0 wt% (w/w); the diarginine or salt, ester, derivative thereof is administered at a dosage of 0.1 µM to 1 M or 0.1-30 g or 0.001 to 90.0 wt% (w/w).

In some embodiments, the composition is administered at least once per day for 1 week to 7 weeks, 2 weeks to 7 weeks, 3 weeks to 7 weeks, and 4 weeks to 7 weeks.

In some embodiments, the method includes oral administration, intravenous injection, intramuscular injection, intraperitoneal injection, topical drip, external use or sublingual administration, and other administration routes.

For example, the acetyltaurine or hydrate, salt, ester, solvate, derivative thereof is administered in a daily dose ranging from 0.05 g to 20 g, 0.05 g to 15 g, 0.1 g to 20 g, 0.3 g to 18 g, 0.5 g to 15 g, 1 g to 20 g, 1 g to 18 g, 1 g to 15 g, 1 g to 10 g, by a single dose or multiple divided doses. In some embodiments, the daily dose ranges from 0.005% to 90%, 0.01% to 85%, 0.05% to 80%, 0.05% to 80%, 0.5% to 75%, 1% to 70%, 2% to 50%, 0.005% to 45%, 0.005% to 40%, 0.005% to 30%, 0.005% to 25%, 0.01% to 40%, 0.01% to 30%, 0.01% to 25%, 0.01% to 15%, 0.05% to 30%, 0.05% to 20%, 0.05% to 10%, 0.1% to 35%, 0.1% to 25%, 0.1% to 10%, 0.2% to 25%, 0.2% to 10%, 0.5% to 9%, 1% to 7%, 2% to 7%, 0.005% to 7%, 0.005% to 9% by weight (w/w). In some embodiments, the daily dose ranges from 0.1 µM to 1 M (e.g., 0.1 µM to 500 µM, 1 µM to 500 µM, 1 µM to 5 mM, 1 µM to 500 mM, 5 µM to 500 µM, 5 µM to 5 mM, 5 µM to 100 mM, 5 µM to 500 mM, 50 µM to 500 µM, 50 µM to 5 mM). The diarginine or salt, ester, derivative thereof is administered in a daily dose ranging from 0.1 g to 30 g, 0.5 g to 30 g, 0.5 g to 25 g, 0.5 g to 20 g, 1 g to 30 g, 1 g to 28 g, 1 g to 25 g, 1 g to 20 g, by a single dose or multiple divided doses. In some embodiments, the daily dose ranges from 0.005% to 90%, 0.01% to 85%, 0.05% to 80%, 0.05% to 80%, 0.5% to 75%, 1% to 70%, 2% to 50%, 0.005% to 45%, 0.005% to 40%, 0.005% to 30%, 0.005% to 25%, 0.01% to 40%, 0.01% to 30%, 0.01% to 25%, 0.01% to 15%, 0.05% to 30%, 0.05% to 20%, 0.05% to 10%, 0.1% to 35%, 0.1% to 25%, 0.1% to 10%, 0.2% to 25%, 0.2% to 10%, 0.5% to 9%, 1% to 7%, 2% to 7%, 0.005% to 7%, 0.005% to 9% by weight (w/w). In some embodiments, the daily dose ranges from 0.1 µM to 1 M (e.g., 0.1 µM to 500 µM, 1 µM to 500 µM, 1 µM to 5 mM, 1 µM to 500 mM, 5 µM to 500 µM, 5 µM to 5 mM, 5 µM to 100 mM, 5 µM to 500 mM, 50 µM to 500 µM, 50 µM to 5 mM).

In some embodiments, the method further comprises administering a component for improving nitric oxide signal or level, which is coenzyme Q10, vitamin E, vitamin C, carnosine, lycopene, creatine antioxidant, arginine, cysteine, resveratrol, taurine, ergothioneine, glutathione.

Another aspect of the present invention relates to use of acetyltaurine or a hydrate, salt, ester, solvate, derivative thereof; or diarginine or a salt, ester, derivative thereof; or a mixture of both in a manufacture of a composition for enhancing or improving muscle function and/or improving exercise performance and/or preventing or alleviating fatigue and/or increasing or improving nitric oxide signal or level.

In some embodiments, the enhancing or improving muscle function includes one or more of the following: alleviating or preventing muscle dysfunction, improving mitochondrial function in muscle, improving insulin resistance in muscle, increasing muscle mass, increasing muscle strength and endurance, improving glucose transport and metabolism in skeletal muscle, alleviating or preventing muscle injury, and reducing muscle fatigue; the improving exercise performance includes one or more of the following: increasing running distance, increasing exercise time, increasing running speed, improving exercise endurance or explosive force, reducing inflammation and muscle fatigue after exercise, and improving muscle contraction during exercise; the preventing or alleviating fatigue includes preventing physical fatigue after exercise and accelerating recovery after exercise.

In some embodiments, the method or composition of the present invention can increase the level of nitrite and/or cyclic guanosine monophosphate.

In some embodiments, the composition enhances or improves muscle function and/or improves exercise performance and/or prevents or alleviates fatigue by improving nitric oxide level or signal, or increasing the level of cyclic guanosine monophosphate (cGMP), or increasing the level of nitrite.

In some embodiments, compared with subject not receiving composition, the enhancement or improvement in muscle function or exercise performance and/or the prevention or alleviation of fatigue in subject of administration group is more than 10%, 30%, 40%, 50%, 60%, 70% or 90%.

In some embodiments, compared with exercise group not receiving administration, the exercise time of administration group increases more than 10%, 30%, 45%, 50%, 60%, 70% or 90%. Compared with exercise group not receiving administration, the running distance of administration group increases more than 10%, 20%, 30%, 35% or 50%. Compared with exercise group not receiving administration, the urea nitrogen level in administration group decreases more than 10%, 15%, 20%, 30% or 40%. Compared with exercise group not receiving administration, the liver glycogen level in administration group increases more than 10%, 15%, 20%, 30% or 40%.

In some embodiments, compared with control subject not receiving administration, nitric oxide signal or level in subject of administration group increases or improves more than 10%, 30%, 40%, 50%, 60%, 70%, 90%, 120%, 150% or 180%.

In some embodiments, the acetyltaurine or hydrate, salt, ester, solvate, derivative thereof; or the diarginine or salt, ester, derivative thereof; or the mixture of both, is prepared into solid preparation or liquid preparation.

In some embodiments, the acetyltaurine or hydrate, salt, ester, solvate, derivative thereof; or the diarginine or salt, ester, derivative thereof; or the mixture of both, is in the form of lotion, paste, suppository, tablet, pill, granule, powder, film, capsule, beverage, aerosol, elixir, tincture, tonic, liquid suspension, syrup.

In some embodiments, the acetyltaurine or hydrate, salt, ester, solvate, derivative thereof is administered at a dosage of 0.1 µM to 1 M or 0.05-20 g or 0.001 to 90.0 wt% (w/w); the diarginine or salt, ester, derivative thereof is administered at a dosage of 0.1 µM to 1 M or 0.1-30 g or 0.001 to 90.0 wt% (w/w).

In some embodiments, the acetyltaurine or hydrate, salt, ester, solvate, derivative thereof is administered in a daily dose ranging from 0.05 g to 20 g, 0.05 g to 15 g, 0.1 g to 20 g, 0.3 g to 18 g, 0.5 g to 15 g, 1 g to 20 g, 1 g to 18 g, 1 g to 15 g, 1 g to 10 g, by a single dose or multiple divided doses. In some embodiments, The daily dose ranges from 0.005% to 90%, 0.01% to 85%, 0.05% to 80%, 0.05% to 80%, 0.5% to 75%, 1% to 70%, 2% to 50%, 0.005% to 45%, 0.005% to 40%, 0.005% to 30%, 0.005% to 25%, 0.01% to 40%, 0.01% to 30%, 0.01% to 25%, 0.01% to 15%, 0.05% to 30%, 0.05% to 20%, 0.05% to 10%, 0.1% to 35%, 0.1% to 25%, 0.1% to 10%, 0.2% to 25%, 0.2% to 10%, 0.5% to 9%, 1% to 7%, 2% to 7%, 0.005% to 7%, 0.005% to 9% by weight (w/w). In some embodiments, the daily dose ranges from 0.1 µM to 1 M (e.g., 0.1 µM to 500 µM, 1 µM to 500 µM, 1 µM to 5 mM, 1 µM to 500 mM, 5 µM to 500 µM, 5 µM to 5 mM, 5 µM to 100 mM, 5 µM to 500 mM, 50 µM to 500 µM, 50 µM to 5 mM). The diarginine or salt, ester, derivative thereof is administered in a daily dose ranging from 0.1 g to 30 g, 0.5 g to 30 g, 0.5 g to 25 g, 0.5 g to 20 g, 1 g to 30 g, 1 g to 28 g, 1 g to 25 g, 1 g to 20 g, by a single dose or multiple divided doses. In some embodiments, the daily dose ranges from 0.005% to 90%, 0.01% to 85%, 0.05% to 80%, 0.05% to 80%, 0.5% to 75%, 1% to 70%, 2% to 50%, 0.005% to 45%, 0.005% to 40%, 0.005% to 30%, 0.005% to 25%, 0.01% to 40%, 0.01% to 30%, 0.01% to 25%, 0.01% to 15%, 0.05% to 30%, 0.05% to 20%, 0.05% to 10%, 0.1% to 35%, 0.1% to 25%, 0.1% to 10%, 0.2% to 25%, 0.2% to 10%, 0.5% to 9%, 1% to 7%, 2% to 7%, 0.005% to 7%, 0.005% to 9% by weight (w/w). In some embodiments, the daily dose ranges from 0.1 µM to 1 M (e.g., 0.1 µM to 500 µM, 1 µM to 500 µM, 1 µM to 5 mM, 1 µM to 500 mM, 5 µM to 500 µM, 5 µM to 5 mM, 5 µM to 100 mM, 5 µM to 500 mM, 50 µM to 500 µM, 50 µM to 5 mM).

Another aspect of the present invention relates to a composition comprising acetyltaurine or a hydrate, salt, ester, solvate, derivative thereof; or diarginine or a salt, ester, derivative thereof; or a mixture of both, where the composition is used for enhancing or improving muscle function and/or improving exercise performance and/or preventing or alleviating fatigue and/or increasing or improving nitric oxide signal or level in a subject.

In some embodiments, the enhancing or improving muscle function includes one or more of the following: alleviating or preventing muscle dysfunction, improving mitochondrial function in muscle, improving insulin resistance in muscle, increasing muscle mass, increasing muscle strength and endurance, improving glucose transport and metabolism in skeletal muscle, alleviating or preventing muscle injury, and reducing muscle fatigue; the improving exercise performance includes one or more of the following: increasing running distance, increasing exercise time, increasing running speed, improving exercise endurance or explosive force, reducing inflammation and muscle fatigue after exercise, and improving muscle contraction during exercise; the preventing or alleviating fatigue includes preventing physical fatigue after exercise and accelerating recovery after exercise.

In some embodiments, the composition of the present invention can increase the level of nitrite and/or cyclic guanosine monophosphate.

In some embodiments, the composition enhances or improves muscle function and/or improves exercise performance and/or prevents or alleviates fatigue by improving nitric oxide level or signal, or increasing the level of cyclic guanosine monophosphate (cGMP), or increasing the level of nitrite.

In some embodiments, compared with composition not comprising acetyltaurine or a hydrate, salt, ester, solvate, derivative thereof; or diarginine or a salt, ester, derivative thereof; or a mixture of both, the enhancement or improvement in muscle function or exercise performance and/or the prevention or alleviation of fatigue in subject administered with the composition comprising acetyltaurine or a hydrate, salt, ester, solvate, derivative thereof; or diarginine or a salt, ester, derivative thereof; or a mixture of both is more than 10%, 30%, 40%, 50%, 60%, 70% or 90%.

In some embodiments, compared with exercise group not receiving administration, the exercise time of administration group increases more than 10%, 30%, 45%, 50%, 60%, 70% or 90%. Compared with exercise group not receiving administration, the running distance of administration group increases more than 10%, 20%, 30%, 35% or 50%. Compared with exercise group not receiving administration, the urea nitrogen level in administration group decreases more than 10%, 15%, 20%, 30% or 40%. Compared with exercise group not receiving administration, the liver glycogen level in administration group increases more than 10%, 15%, 20%, 30% or 40%.

In some embodiments, compared with control subject not receiving administration, nitric oxide signal or level in subject of administration group increases or improves more than 10%, 30%, 40%, 50%, 60%, 70%, 90%, 120%, 150% or 180%.

In some embodiments, the composition is in the form of lotion, paste, suppository, tablet, pill, granule, powder, film, capsule, beverage, aerosol, elixir, tincture, tonic, liquid suspension, syrup.

In some embodiments, in the composition, the amount of the acetyltaurine or hydrate, salt, ester, solvate, derivative thereof is 0.1 µM to 1 M or 0.05-20 g or 0.001 to 50.0 wt% (w/w); the amount of the diarginine or salt, ester, derivative thereof is 0.1 µM to 1 M or 0.1-30 g or 0.001 to 90.0 wt% (w/w).

In some embodiments, the composition further comprises other component for improving nitric oxide signal or level. The component for improving nitric oxide signal or level includes coenzyme Q10, vitamin E, vitamin C, carnosine, lycopene, creatine antioxidant, arginine, cysteine, resveratrol, taurine, ergothioneine, glutathione.

Another aspect of the present invention relates to a method for increasing or improving the level of nitrite and/or cyclic guanosine monophosphate, where the method comprises: administering acetyltaurine or a hydrate, salt, ester, solvate, derivative thereof; or diarginine or a salt, ester, derivative thereof; or a mixture of both to a subject.

In some embodiments, an effective amount of acetyltaurine or a hydrate, salt, ester, solvate, derivative thereof; or diarginine or a salt, ester, derivative thereof; or a mixture of both, is administered to the subject.

In some embodiments, compared with control subject not receiving administration, the level of nitrite and/or cyclic guanosine monophosphate in subject of administration group increases or improves more than 10%, 30%, 40%, 50%, 60%, 70% or 90%.

In some embodiments, the subject is human or mammal.

In some embodiments, the acetyltaurine or hydrate, salt, ester, solvate, derivative thereof; or the diarginine or salt, ester, derivative thereof; or the mixture of both is formulated as nutritional product, dietary supplement, food or beverage.

In some embodiments, the acetyltaurine or hydrate, salt, ester, solvate, derivative thereof; or the diarginine or salt, ester, derivative thereof; or the mixture of both, is prepared into solid preparation or liquid preparation.

In some embodiments, the acetyltaurine or hydrate, salt, ester, solvate, derivative thereof; or the diarginine or salt, ester, derivative thereof; or the mixture of both, is in the form of lotion, paste, suppository, tablet, pill, granule, powder, film, capsule, beverage, aerosol, elixir, tincture, tonic, liquid suspension, syrup.

In some embodiments, the acetyltaurine or hydrate, salt, ester, solvate, derivative thereof is administered at a dosage of 0.1 µM to 1 M or 0.105-20 g or 0.001 to 90.0 wt% (w/w); the diarginine or salt, ester, derivative thereof is administered at a dosage of 0.1 µM to 1 M or 0.1-30 g or 0.001 to 90.0 wt% (w/w).

Another aspect of the present invention relates to use of acetyltaurine or a hydrate, salt, ester, solvate, derivative thereof; or diarginine or a salt, ester, derivative thereof; or a mixture of both in a manufacture of a composition for increasing or improving the level of nitrite and/or cyclic guanosine monophosphate.

In some embodiments, compared with control subject not receiving administration, the level of nitrite and/or cyclic guanosine monophosphate in subject of administration group increases or improves more than 10%, 30%, 40%, 50%, 60%, 70%, 90%, 120%, 150% or 180%.

In some embodiments, the acetyltaurine or hydrate, salt, ester, solvate, derivative thereof; or the diarginine or salt, ester, derivative thereof; or the mixture of both, is prepared into solid preparation or liquid preparation.

In some embodiments, the acetyltaurine or hydrate, salt, ester, solvate, derivative thereof; or the diarginine or salt, ester, derivative thereof; or the mixture of both, is in the form of lotion, paste, suppository, tablet, pill, granule, powder, film, capsule, beverage, aerosol, elixir, tincture, tonic, liquid suspension, syrup.

In some embodiments, the acetyltaurine or hydrate, salt, ester, solvate, derivative thereof has a dosage of 0.1 µM to 1 M or 10.05-20 g or 0.001 to 90.0 wt% (w/w); the diarginine or salt, ester, derivative thereof is administered at a dosage of 0.1 µM to 1 M or 0.1-30 g or 0.001 to 90.0 wt% (w/w).

The method of administering acetyltaurine or a hydrate, salt, ester, solvate, derivative thereof; or diarginine or a salt, ester, derivative thereof; or a mixture of both; or a composition comprising the same to a subject of the present invention can be used for enhancing or improving muscle function or exercise performance and/or preventing or alleviating fatigue and/or increasing or improving nitric oxide signal or level; increasing or improving the level of nitrite and/or cyclic guanosine monophosphate in the subject. It plays a vital role in the normal function of cardiovascular system, nerve, lung, gastrointestinal tract, kidney and immune system.

### BRIEF DESCRIPTION OF THE DRAWINGS

Figure 1 shows swimming times of groups 2-4 that performed exercise.
Figure 2 shows running distances of groups 2-4 that performed exercise.
Figure 3 shows blood urea nitrogen levels of groups 1-4.
Figure 4 shows liver glycogen levels of groups 1-4.
Figure 5 shows muscle glycogen levels of groups 1-4.
Figure 6 shows a histogram of load-bearing swimming exhaustion time of mice in EX group and ATR group.
Figure 7 shows a histogram of running exhaustion distance of EX group and ATR group mice.
Figure 8 shows a histogram of cyclic guanosine monophosphate (cGMP) content in serum of EX group and ATR group mice.
Figure 9 shows a histogram of nitric oxide (NO) content in serum of mice in groups 5-8.
Figure 10 shows a histogram of lactic acid (LA) content in serum of mice in EX group and ATR group.
Figure 11 shows a histogram of urea nitrogen (BUN) content in serum of EX group and ATR group mice.
Figure 12 shows a histogram of liver glycogen (LG) content in EX group and ATR group mice.
Figure 13 shows the area under curve (AUC) of NO in two groups of mice.
Figure 14 shows the area under curve (AUC) of cGMP in two groups of mice.

### DETAILED DESCRIPTION OF THE EMBODIMENTS

While the present invention will be described in conjunction with the embodiments, it will be understood that they are not intended to limit the present invention to these embodiments. To the contrary, the present invention is intended to cover alternatives, modifications and equivalents, which may be included within the spirit and scope of the present invention as defined by the claims. Furthermore, in the detailed description of the present invention, numerous specific details are set forth in order to provide a thorough understanding of the present invention. However, it will be obvious to one of ordinary skill in the art that the present invention may be practiced without these specific details. In other instances, well known methods, procedures, components, and other features have not been described in detail.

As used herein, the term "or" is meant to include both "and" and "or." In other words, the term "or" may also be replaced with "and/or."

As used herein, the singular forms "a," "an" and "the" are intended to include the plural forms as well, unless the context clearly indicates otherwise.

As used herein, the term "comprise" or "include" and their conjugations, refer to a situation where said terms are used in their non-limiting sense to mean that items following the word are included, but items not specifically mentioned are not excluded. It also encompasses the more limiting verb 'to consist essentially of' and 'to consist of'.

As used herein, the terms "subject" or "individual" are used interchangeably to refer to any subject to whom the disclosed methods and compositions can be applied or administered. The subject may have a disease or disorder, but the subject does not need to be sick to benefit from the disclosed methods and compositions. Any subject can take the disclosed composition or become a recipient of the disclosed method. As used herein, the term "subject" refers to animals (for example, birds, reptiles and mammals). In some embodiments, the subject can be mammals including non-primates (e.g., camels, donkeys, zebras, cows, horses, cats, dogs, rats and mice) and primates (e.g., monkeys, chimpanzees and humans). In certain embodiments, the subject may be a non-human mammal. In other embodiments, the subject may be a human.

The term "administer", "administering", or "administration", as used in the present invention, refers to either directly administering a compound or pharmaceutically acceptable salt of the compound or a composition to a subject, or administering a prodrug derivative or analog of the compound or pharmaceutically acceptable salt of the compound or composition to the subject, which can form an equivalent amount of active compound within the subject's body.

Depending on the particular acetyltaurine-containing formulation and form, contemplated methods include an administration of an appropriate amount of acetyltaurine or acetyltaurine substitute every day. This amount per day may be administered at once or in multiple doses. Typically, an effective amount of acetyltaurine or acetyltaurine derivative in the acetyltaurine-containing composition to be administered at once or in multiple doses per day is of or between 0.05 gram (g) to 20 g. One or more doses may be administered once a day for any period of time. For example, the effective dose may be administered each day for one day, a few days, multiple days, or on a daily basis indefinitely. Acetyltaurine can be administered for 7 days or more in a cycle, depending on the desired effect. More typically, the amount of acetyltaurine or acetyltaurine derivative to be administered each day is 0.05 g, 0.1 g, 0.2 g, 0.3 g, 0.5 g, 0.6 g, 0.8 g, 0.9 g, 1 g, 1.2 g, 1.4 g, 1.5 g, 2 g, 3.5 g, 4 g, 5 g, 8 g, 10 g, 12 g, 15 g, 18 g, 20 g. More typically, the amount of acetyltaurine or acetyltaurine derivative to be administered each day is 0.2 g to 3 g. Most typically, the amount of acetyltaurine or acetyltaurine derivative to be administered each day is 1 g.

An effective amount of acetyltaurine or acetyltaurine derivative in the acetyltaurine-containing composition to be administered at once or in multiple doses per day is 0.001% to 50.0% by weight or between 0.001% to 90.0% by weight (w/w). One or more doses may be administered once a day for any period of time. For example, the effective dose may be administered each day for one day, a few days, multiple days, or on a daily basis indefinitely. Acetyltaurine can be administered for 7 days or more in a cycle, depending on the desired effect. More typically, the amount of acetyltaurine or acetyltaurine derivative to be administered each day is 0.005%, 0.01%, 0.05%, 0.1%, 0.2%, 0.5%, 1%, 2%, 3%, 4%, 5%, 7%, 9%, 10%, 12%, 15%, 16%, 18%, 20%, 25%, 30%, 35%, 40%, 45%, 50%, 60%, 70%, 80%, 90% by weight (w/w). The amount of acetyltaurine or acetyltaurine derivative administered at once or in multiple doses per day may be 0.1 µM to 1 M (e.g., 0.1 µM to 500 µM, 1 µM to 500 µM, 1 µM to 5 mM, 1 µM to 100 mM, 1 µM to 500 mM, 5 µM to 500 µM, 5 µM to 5 mM, 5 µM to 100 mM, 5 µM to 500 mM, 50 µM to 500 µM, 50 µM to 5 mM). One dose or multiple doses can be administered at any time of day. For example, the effective dose may be administered each day for one day, a few days, multiple days, or on a daily basis indefinitely.

Depending on the particular diarginine-containing formulation and form, contemplated methods include an administration of an appropriate amount of diarginine or a salt, ester, derivative thereof every day. This amount per day may be administered at once or in multiple doses. Typically, an effective amount of diarginine or diarginine derivative in the diarginine-containing composition to be administered at once or in multiple doses per day is of or between 0.1 gram (g) to 30 g. One or more doses may be administered once a day for any period of time. For example, the effective dose may be administered each day for one day, a few days, multiple days, or on a daily basis indefinitely. Diarginine can be administered for 7 days or more in a cycle, depending on the desired effect. More typically, the amount of diarginine or diarginine derivative to be administered each day is 0.1 g, 0.2 g, 0.3 g, 0.5 g, 0.7g, 0.9 g, 1 g, 1.2 g, 1.5 g, 3 g, 5 g, 8 g, 10 g, 12 g, 15 g, 18 g, 20 g, 25 g, 30 g. More typically, the amount of diarginine or diarginine derivative to be administered each day is 1 g to 30 g. Most typically, the amount of diarginine or diarginine derivative to be administered each day is 8 g.

An effective amount of diarginine or diarginine derivative in the diarginine-containing composition to be administered at once or in multiple doses per day is 0.001% to 50.0% by weight or between 0.001% to 90.0% by weight (w/w). One or more doses may be administered once a day for any period of time. For example, the effective dose may be administered each day for one day, a few days, multiple days, or on a daily basis indefinitely. Diarginine can be administered for 7 days or more in a cycle, depending on the desired effect. More typically, the amount of diarginine or diarginine derivative to be administered each day is 0.005%, 0.01%, 0.05%, 0.1%, 0.2%, 0.5%, 1%, 2%, 3%, 4%, 5%, 7%, 9%, 10%, 12%, 15%, 16%, 18%, 20%, 25%, 30%, 35%, 40%, 45%, 50%, 60%, 70%, 80%, 90% by weight (w/w). The amount of diarginine or diarginine derivative administered at once or in multiple doses per day may be 0.1 µM to 1 M (e.g., 0.1 µM to 500 µM, 1 µM to 500 µM, 1 µM to 5 mM, 1 µM to 100 mM, 1 µM to 500 mM, 5 µM to 500 µM, 5 µM to 5 mM, 5 µM to 100 mM, 5 µM to 500 mM, 50 µM to 500 µM, 50 µM to 5 mM). One dose or multiple doses can be administered at any time of day. For example, the effective dose may be administered each day for one day, a few days, multiple days, or on a daily basis indefinitely.

The amount of acetyltaurine or a hydrate, salt, ester, solvate, derivative thereof; or diarginine or a salt, ester, derivative thereof; or a mixture of both; or composition comprising them can be selected as required.

Multiple forms and formulations of the composition comprising acetyltaurine or diarginine or their mixture are contemplated. Compositions will be formulated as a nutritional or dietary supplement, (medical) food item, skin care product, in liquid or solid form, comprising acetyltaurine or diarginine or their mixture, and may optionally also comprise a nutritionally or pharmaceutically acceptable carrier. For oral administration, the composition of the present invention can be in any suitable form, including solution, tablet, gel capsule, capsule or alternative nutritious food or nutritional supplement.

The technical features, implementation methods and beneficial effects of the present invention will be further described in detail with specific implementations. Unless otherwise specified, the materials and reagents described in the following examples are all common commercial products and can be purchased in the market.

### Examples

### Example 1

Thirty-two Kunming mice were randomly divided into normal control group (blank, no exercise, group 1), exercise group (blank, group 2), arginine exercise group (700 mg/kg, group 3) and diarginine exercise group (350 mg/kg, group 4), with 8 mice in each group. Gavage was continued for 32 days, and weight was recorded every week. Animals in groups 2, 3 and 4 underwent load-bearing swimming endurance test 30 minutes after administration on the 28th day; underwent treadmill endurance test on the 30th day; swam without load for 90 minutes 30 minutes after administration on the 32nd day, and blood and tissue samples were collected immediately after swimming.

Blood was collected through retroorbital approach immediately after swimming without load for 90 minutes on the 32nd day. After blood was allowed to stand at room temperature for 4 hours, it was centrifuged at 1500xg at 4°C for 10 min, and all serum was transferred to a cryogenic tube for storage at -80°C. Liver and gastrocnemius-soleus muscle were removed and weighed.

Swimming endurance test: On the 28th day of administration in each group, load-bearing swimming endurance test was carried out. Lead wire was wrapped around tail 30 minutes after administration, and the weight of lead wire was 5% of the body weight. The time from animal entering water to the time when head completely entered water for 8 s and could not surface was recorded by a stopwatch. Swimming time of each mouse was recorded.

Treadmill endurance test: Muscle endurance test was conducted on the 30th day of administration in each group. Muscle endurance test was accomplished on an electric treadmill with a speed range of 5 to 50 cm/s and an inclination of 10 degrees. Running uphill involves concentric muscle contraction, which increases muscle work and leads to faster fatigue compared with running on the flat ground. Belt speed started from about 15 cm/s and increases by 5 cm/s every 2 minutes until it reached a speed of 50 cm/s. Animals ran treadmill tests until they were exhausted. The point of exhaustion was defined at the time when the animal fell into the shock zone. Movement distance (cm) was measured as a sign of exercise performance.

Clinical biochemical survey: After swimming without load for 90 minutes on the 32nd day of administration, serum was collected for clinical biochemical index analysis, including nitric oxide (NO), lactic acid (LA) and urea nitrogen (BUN). Cyclic guanosine monophosphate (cGMP) was detected using standard ELISA kit. In addition, part of liver and gastrocnemius-soleus muscle were taken for glycogen content determination.

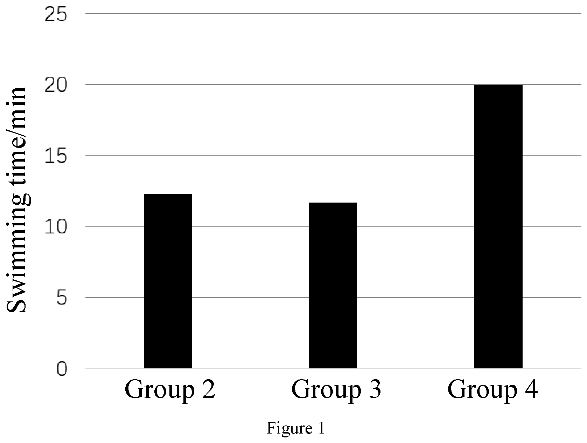

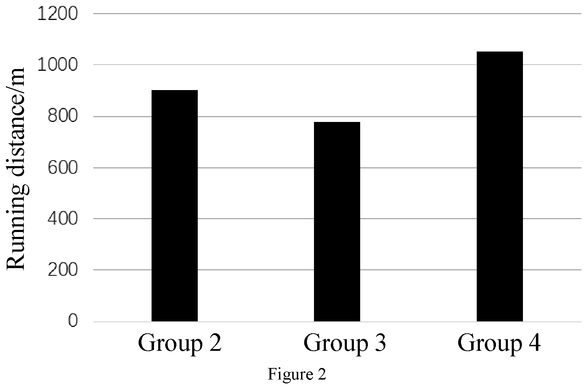

Figure 1 shows swimming times of groups 2-4 that performed exercise. Swimming time of group 4 is 8.3 minutes longer than that of group 3, and exercise time is increased by 70.9%; while exercise time of group 4 is increased by 62.6% compared with group 2; while exercise time of group 3 is decreased compared with that of group 2. The mice recovered 2 days after swimming, and running endurance test was performed 30 minutes after administration. Figure 2 shows running distances of groups 2-4 that performed exercise. After 30 days of administration, running distance of group 4 is 275 m longer than that of group 3, and running distance increased by 35.3%; while running distance of group 4 is 154.4 m longer than that of group 2, and running distance is increased by 17.2%; while running distance of group 3 is decreased compared with that of group 2.

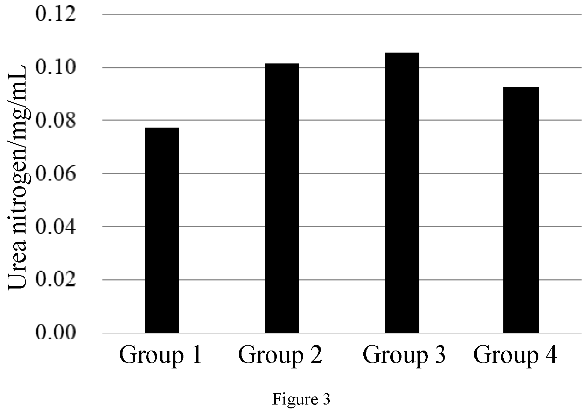

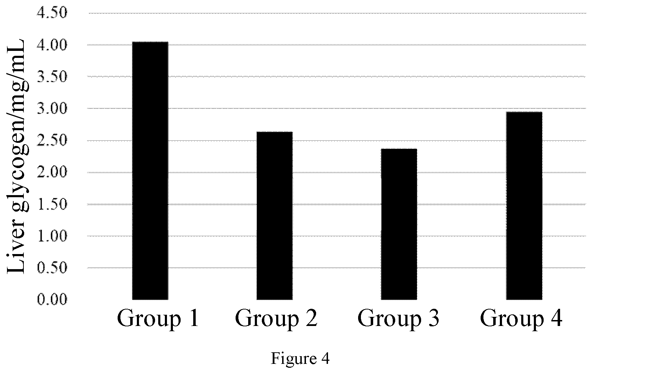

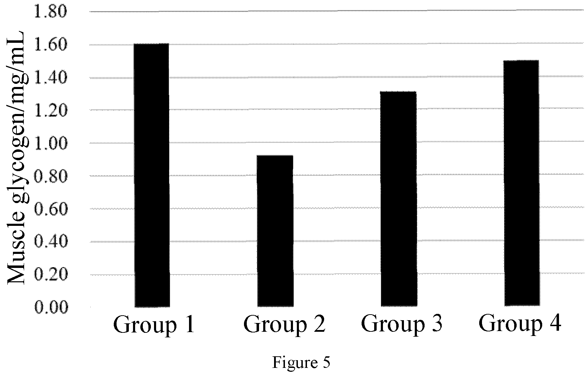

Figure 3 shows blood urea nitrogen levels of groups 1-4. After swimming for 90 minutes on the 32nd day of administration, blood urea nitrogen content of group 4 is significantly lower than that of group 3, which is 18.2% lower; and that of group 4 is 11.7% lower than that of group 2; while blood urea nitrogen level of group 3 is higher than that of group 2. Figure 4 shows liver glycogen levels of groups 1-4. Liver glycogen content of group 4 is higher than that of group 3, which is 24.5% higher; and that of group 4 is 11.9% higher than that of group 2. Figure 5 shows muscle glycogen levels of groups 1-4. Muscle glycogen content of group 4 is higher than that of group 3, which is 13.7% higher; while that of group 4 is 62.0% higher than that of group 2. This shows that glycogen loss in group 4 is obviously reduced, urea nitrogen level is reduced, and increase of lactic acid level after exercise is inhibited. Anti-fatigue indexes include lactic acid, urea nitrogen and glycogen. During exercise, the body consumes energy, and glycogen can be fermented to produce lactic acid, which can provide energy for muscles, so muscle glycogen and lactic acid can reflect muscle function. Swimming time and running distance can reflect exercise performance, and urea nitrogen and liver glycogen can reflect the anti-exercise fatigue effect of the body. The present invention finds that diarginine can obviously reduce muscle fatigue and accelerate recovery after exercise, and the effect is better than arginine.

### Example 2

Forty Kunming mice were randomly divided into normal control group (blank, control group 5), exercise control group (blank, EX group 6), acetyltaurine exercise group (500 mg/kg, ATR group 7) and diarginine exercise group (500 mg/kg, Di-Arg group 8), with 10 mice in each group. Gavage was continued for 28 days, and weight was recorded every week. All mice in exercise groups underwent 5% load-bearing swimming test on the 28th day after administration, and swimming exhaustion time was recorded; underwent treadmill exhaustion experiment 30 days after administration, and running exhaustion distance of mice was recorded; swam without load for 90 minutes on the 32nd day after administration, and blood and tissue samples were collected immediately after swimming. After blood was allowed to stand at room temperature for 4 hours, it was centrifuged at 1500xg at 4°C for 10 min, and all serum was transferred to a cryogenic tube for storage at -80°C. All animals were tested for exercise after 30 minutes of intragastric administration.

Load-bearing swimming test: Lead wire was wrapped around tail 30 minutes after administration, and the weight of lead wire was 5% of the body weight. The time from animal entering water to the time when head completely entered water for 10 s and could not surface was recorded by a stopwatch. Swimming time of each mouse was recorded.

Treadmill endurance test: Muscle endurance test was conducted on the 30th day of administration in each group. Muscle endurance test was accomplished on an electric treadmill with a speed range of 0 to 50 cm/s and an inclination of 10 degrees. Running uphill involves concentric muscle contraction, which increases muscle work and leads to faster fatigue compared with running on the flat ground. Belt speed started from about 15 cm/s and increases by 5 cm/s every 2 minutes until it reached a speed of 50 cm/s. Animals ran treadmill tests until they were exhausted. The point of exhaustion was defined at the time when the animal fell into the shock zone. Movement distance (m) was measured as a sign of exercise performance.

Clinical biochemical survey: After swimming without load for 90 minutes on the 32nd day of administration, serum was collected for clinical biochemical index analysis, including cyclic guanosine monophosphate (cGMP), nitric oxide (NO), lactic acid (LA) and urea nitrogen (BUN). In addition, part of liver was taken for liver glycogen (LG) content determination.

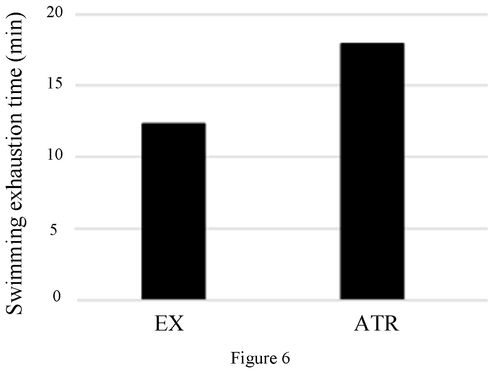

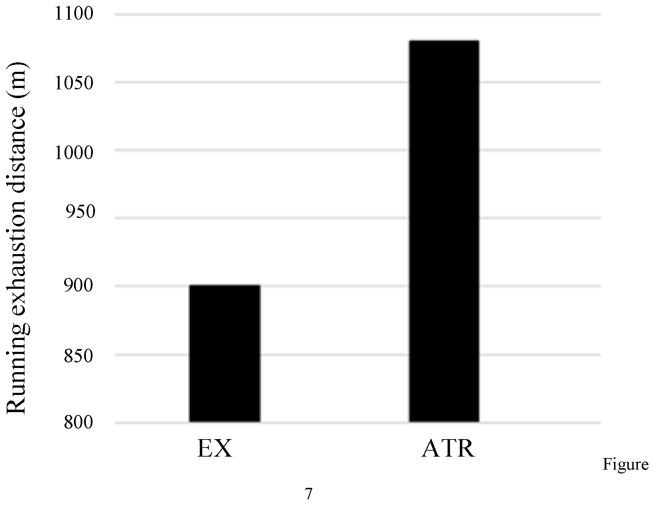

Figure 6 shows a histogram of load-bearing swimming exhaustion time of mice in EX group and ATR group after 28 days of feeding intervention. Results show that compared with exercise control group mice, load-bearing swimming exhaustion time of mice intervened by acetyltaurine is prolonged by 45.5%. Figure 7 shows a histogram of running exhaustion distance of EX group and ATR group mice after 30 days of feeding intervention. Results show that compared with exercise control group mice, running exhaustion distance of mice intervened by acetyltaurine is prolonged by 20.0%.

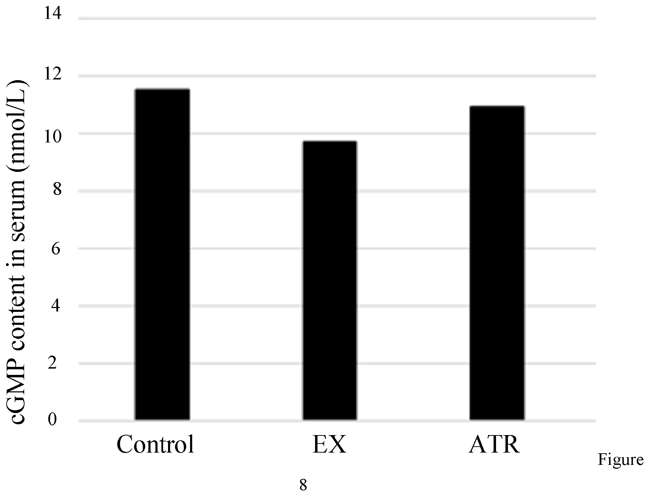

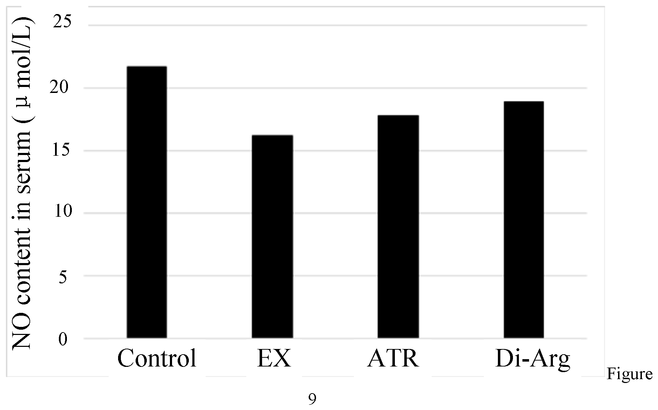

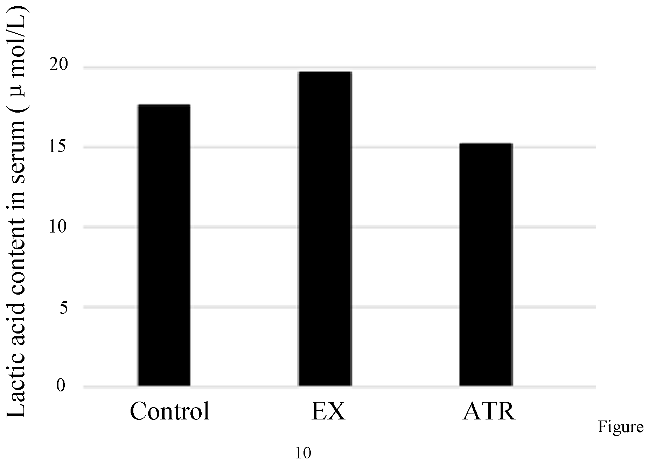

Figure 8 shows a histogram of cyclic guanosine monophosphate (cGMP) content in serum of EX group and ATR group mice after fatigue exercise after 32 days of feeding intervention. Results show that compared with exercise control group mice, the level of cGMP in serum of mice intervened by acetyltaurine is increased by 12.4%. Figure 9 shows a histogram of nitric oxide (NO) content in serum of mice in groups 5-8 after fatigue exercise after 32 days of feeding intervention. Results show that compared with exercise control group, nitric oxide (NO) content in serum of mice intervened by acetyltaurine is increased by 9.8% after exercise; nitric oxide (NO) content in serum of mice intervened by diarginine is increased by 16.7%. Figure 10 shows a histogram of lactic acid (LA) content in serum of mice in EX group and ATR group after fatigue exercise after 32 days of feeding intervention. Results show that compared with exercise control group, acetyltaurine intervention could reduce the accumulation of lactic acid caused by exercise in serum of mice by 22.8%.

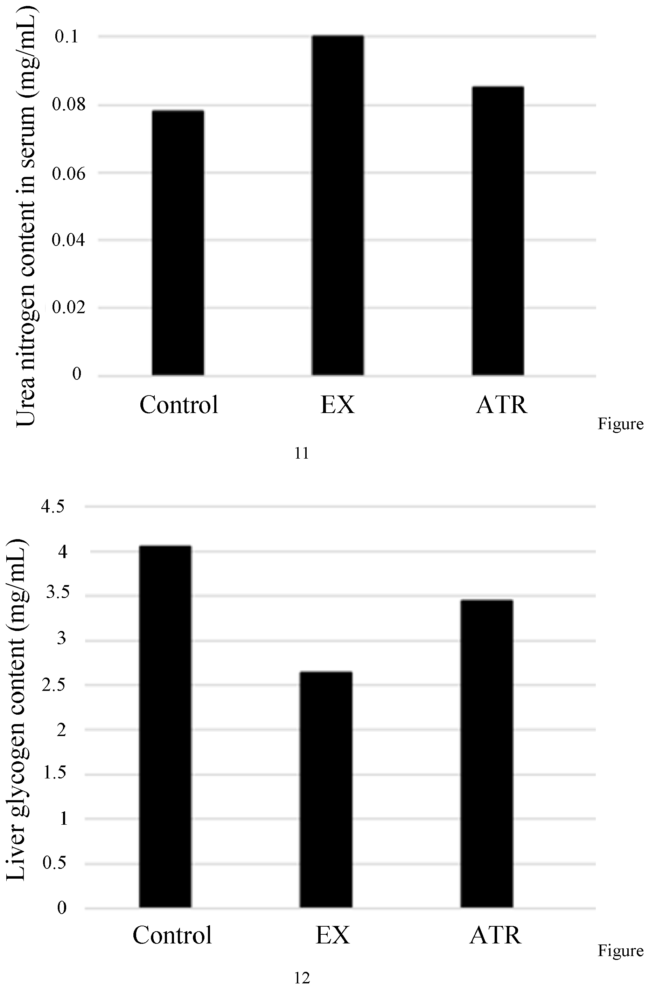

Figure 11 shows a histogram of urea nitrogen (BUN) content in serum of EX group and ATR group mice after fatigue exercise after 32 days of feeding intervention. Results show that compared with exercise control group, acetyltaurine intervention could reduce the accumulation of urea nitrogen during exercise by 15.0%.

Figure 12 shows a histogram of liver glycogen (LG) content in EX group and ATR group mice after fatigue exercise after 32 days of feeding intervention. Results show that fatigue exercise causes liver glycogen consumption, but liver glycogen level is increased by 30.0% in mice intervened by acetyltaurine. Anti-fatigue indexes include lactic acid, urea nitrogen and glycogen. During exercise, the body consumes energy, and glycogen can be fermented to produce lactic acid, which can provide energy for muscles, so muscle glycogen and lactic acid can reflect muscle function. Swimming time and running distance can reflect exercise performance, and urea nitrogen and liver glycogen can reflect the anti-exercise fatigue effect of the body. The present invention finds that acetyltaurine can obviously reduce muscle fatigue and accelerate recovery after exercise.

### Example 3

Sixteen C57 male mice were first adapted to laboratory environment for 7 days. The experimental animals were kept in separate cages, and were free to take food and water. Ambient temperature was 22±1°C, relative humidity was 50±5%, and light/dark circulation alternated for 12 h. After fasting for 16 h, mice were randomly divided into normal control group (blank, group 1) and acetyltaurine group (100 mg/kg, group 2), with 8 mice in each group. Blood samples were taken from eyes of each group at 30 min and 60 min after administration, respectively, to measure NO level. From Table 1, it can be seen that acetyltaurine can obviously improve NO level compared with control group.

**Table 1. NO level in serum**

| **Group** | **NO (µmol/L)** |
|---|---|
| Control | 62.66±22.52 |
| Acetyltaurine | 106.8±8.17* |

Data is mean ± SD of n = 8 mice in each group. * p < 0.05 compared with control group.

### Example 4

Sixteen male SD rats (8 weeks old) freely ingested standard rat food and tap water in a room with temperature control (22±2°C), humidity control (55±5%) and light/dark circulation 12 h.

After fasting for 16 h, they were randomly divided into two groups, one was given solvent and the other was given diarginine (Di-Arg), by gavage. The specific grouping information is shown in Table 2 below. Blood samples were collected from tail of rats at 0, 60, 120 and 180 min after administration. Whole blood was frozen overnight and centrifuged at 3000 rpm for 10 min to obtain serum. NO was measured with serum.

NO²⁻ is easily oxidized by NO in vivo or in aqueous solution. Under acidic conditions, NO²⁻forms diazonium compounds with diazonium sulfonate amine, and further couples with naphthalene ethylenediamine. The product has a characteristic absorption peak at 550 nm, and the light absorption value of the product can be determined. Serum cGMP was detected by ELISA kit, and the concentration of cGMP in blood was calculated according to standard curve.

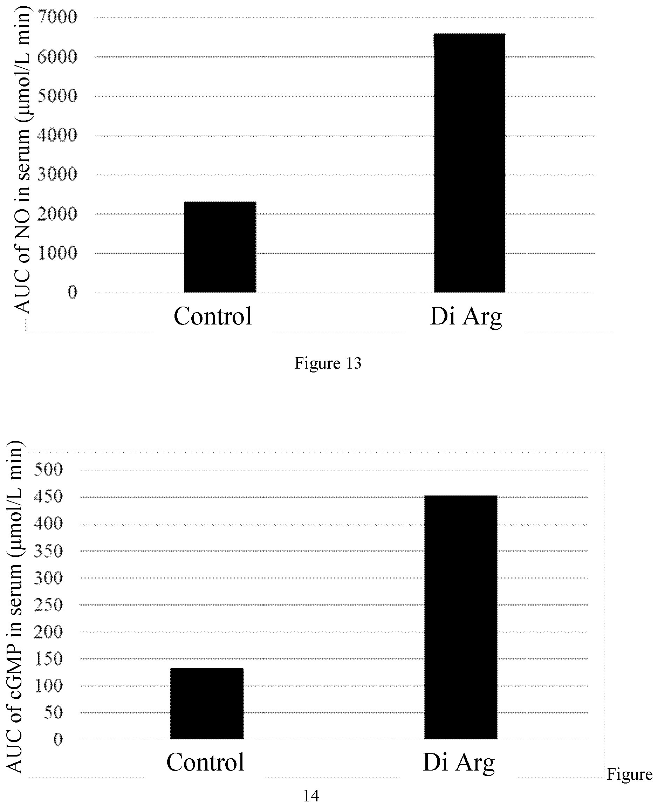

Figure 13 shows the area under curve (AUC) of NO. According to the results of Figure 13, diarginine can significantly promote the synthesis of nitric oxide (NO), which is 183% higher than that of the control group. Figure 14 shows the area under curve (AUC) of cGMP. According to AUC data, the AUC of diarginine group is 454 µmol/L*min, which is 241% higher than that of the control group.

**Table 2. Experimental grouping method**

| **Group** | **Number** | **Sample** | **Dosage in rat** | **Gavage volume** |
|---|---|---|---|---|
| Control | 8 | Saline | / | 10 mL/kg |
| Di-arg | 8 | Di-arg | 238 mg/kg | 10 mL/kg |

To sum up, the method or composition of the present invention can increase or improve nitric oxide signal or level, or improve the level of cyclic guanosine monophosphate, or improve the level of nitrite; enhance or improve muscle function and/or improve exercise performance and/or prevent or alleviate fatigue. Specifically, it can alleviate or prevent muscle dysfunction, improve mitochondrial function in muscle, improve insulin resistance in muscle, increase muscle mass, increase muscle strength and endurance, improve glucose transport and metabolism in skeletal muscle, alleviate or prevent muscle injury, and reduce muscle fatigue; increase running and/or swimming distance, increase exercise time, increase running and/or swimming speed, improve exercise endurance or explosive force, reduce inflammation and muscle fatigue after exercise, and improve muscle contraction during exercise; prevent physical fatigue after exercise and accelerate recovery after exercise.

The above are only the preferred embodiments of the present invention, and are not intended to limit the present invention. Those skilled in the art can make many changes, modifications, substitutions and variations on these embodiments without departing from the principles and purposes of the present invention, and the scope of the present invention is defined by the claims and their equivalents.

## Claims

1. A method for enhancing or improving muscle function and/or improving exercise performance and/or preventing or alleviating fatigue and/or increasing or improving nitric oxide signal or level, **characterized in that** the method comprises: administering acetyltaurine or a hydrate, salt, ester, solvate, or derivative thereof, or diarginine or a salt, ester, or derivative thereof, or a mixture of both to a subject.

2. The method of claim 1, **characterized in that** the enhancing or improving muscle function comprises one or more of the following: alleviating or preventing muscle dysfunction, improving mitochondrial function in muscle, improving insulin resistance in muscle, increasing muscle mass, increasing muscle strength and endurance, improving glucose transport and metabolism in skeletal muscle, alleviating or preventing muscle injury, and reducing muscle fatigue; the improving exercise performance comprises one or more of the following: increasing running and/or swimming distance, increasing exercise time, increasing running and/or swimming speed, improving exercise endurance or explosive force, reducing inflammation and muscle fatigue after exercise, and improving muscle contraction during exercise; the preventing or alleviating fatigue comprises preventing physical fatigue after exercise and accelerating recovery after exercise.

3. The method of claim 1 or 2, **characterized in that** the method increases the level of nitrite and/or cyclic guanosine monophosphate.

4. The method of claim 1 or 2, **characterized in that** the method enhances or improves muscle function and/or improves exercise performance and/or prevents or alleviates fatigue by improving nitric oxide level or signal, or increasing the level of cyclic guanosine monophosphate, or increasing the level of nitrite.

5. The method of any one of claims 1-4, **characterized in that** compared with a control subject not receiving administration, the enhancement or improvement in muscle function or exercise performance and/or the prevention or alleviation of fatigue in a subject of administration group is more than 10%, 30%, 40%, 50%, 60%, 70% or 90%.

6. The method of any one of claims 1-4, **characterized in that** compared with a control subject not receiving administration, nitric oxide signal or level in a subject of administration group increases or improves more than 10%, 30%, 40%, 50%, 60%, 70%, 90%, 120%, 150% or 180%.

7. The method of any one of claims 1-6, **characterized in that** the subject is human or mammal.

8. The method of any one of claims 1-7, **characterized in that** the acetyltaurine or hydrate, salt, ester, solvate, derivative thereof; or the diarginine or salt, ester, derivative thereof; or the mixture of both, is prepared into a solid preparation or a liquid preparation.

9. The method of any one of claims 1-8, **characterized in that** the acetyltaurine or hydrate, salt, ester, solvate, derivative thereof; or the diarginine or salt, ester, derivative thereof; or the mixture of both, is in a form of lotion, paste, suppository, tablet, pill, granule, powder, film, capsule, beverage, aerosol, elixir, tincture, tonic, liquid suspension, syrup.

10. The method of any one of claims 1-9, **characterized in that** the acetyltaurine or hydrate, salt, ester, solvate, derivative thereof is administered at a dosage of 0.1 µM to 1 M or 0.05-20 g or 0.001 to 90.0 wt% (w/w); the diarginine or salt, ester, derivative thereof is administered at a dosage of 0.1 µM to 1 M or 0.1-30 g or 0.001 to 90.0 wt% (w/w).

11. The method of any one of claims 1-10, **characterized in that** the method further comprises administering a component for improving nitric oxide signal or level, which is coenzyme Q10, vitamin E, vitamin C, carnosine, lycopene, creatine antioxidant, arginine, cysteine, resveratrol, taurine, ergothioneine, glutathione.

12. Use of acetyltaurine or a hydrate, salt, ester, solvate, or derivative thereof, or diarginine or a salt, ester, or derivative thereof, or a mixture of both in a manufacture of a composition for enhancing or improving muscle function and/or improving exercise performance and/or preventing or alleviating fatigue and/or increasing or improving nitric oxide signal or level.

13. The use of claim 12, **characterized in that** the enhancing or improving muscle function comprises one or more of the following: alleviating or preventing muscle dysfunction, improving mitochondrial function in muscle, improving insulin resistance in muscle, increasing muscle mass, increasing muscle strength and endurance, improving glucose transport and metabolism in skeletal muscle, alleviating or preventing muscle injury, and reducing muscle fatigue; the improving exercise performance comprises one or more of the following: increasing running and/or swimming distance, increasing exercise time, increasing running and/or swimming speed, improving exercise endurance or explosive force, reducing inflammation and muscle fatigue after exercise, and improving muscle contraction during exercise; the preventing or alleviating fatigue comprises preventing physical fatigue after exercise and accelerating recovery after exercise.

14. The use of claim 12 or 13, **characterized in that** the composition increases the level of nitrite and/or cyclic guanosine monophosphate.

15. The use of claim 12 or 13, **characterized in that** the composition enhances or improves muscle function and/or improves exercise performance and/or prevents or alleviates fatigue by improving nitric oxide level or signal, or increasing the level of cyclic guanosine monophosphate, or increasing the level of nitrite.

16. The use of any one of claims 12-15, **characterized in that** compared with a control subject not receiving administration, the enhancement or improvement in muscle function or exercise performance and/or the prevention or alleviation of fatigue in a subject of administration group is more than 10%, 30%, 40%, 50%, 60%, 70% or 90%.

17. The use of any one of claims 12-15, **characterized in that** compared with a control subject not receiving administration, nitric oxide signal or level in a subject of administration group increases or improves more than 10%, 30%, 40%, 50%, 60%, 70%, 90%, 120%, 150% or 180%.

18. The use of any one of claims 12-17, **characterized in that** the acetyltaurine or hydrate, salt, ester, solvate, derivative thereof; or the diarginine or salt, ester, derivative thereof; or the mixture of both, is prepared into a solid preparation or a liquid preparation.

19. The use of any one of claims 12-18, **characterized in that** the acetyltaurine or hydrate, salt, ester, solvate, derivative thereof; or the diarginine or salt, ester, derivative thereof; or the mixture of both, is in a form of lotion, paste, suppository, tablet, pill, granule, powder, film, capsule, beverage, aerosol, elixir, tincture, tonic, liquid suspension, syrup.

20. The use of any one of claims 12-19, **characterized in that** the acetyltaurine or hydrate, salt, ester, solvate, derivative thereof is administered at a dosage of 0.1 µM to 1 M or 0.05-20 g or 0.001 to 50.0 wt% (w/w); the diarginine or salt, ester, derivative thereof is administered at a dosage of 0.1 µM to 1 M or 0.1-30 g or 0.001 to 90.0 wt% (w/w).

21. A composition, **characterized by** comprising acetyltaurine or a hydrate, salt, ester, solvate, derivative thereof; or diarginine or a salt, ester, derivative thereof; or a mixture of both, wherein the composition is used for enhancing or improving muscle function and/or improving exercise performance and/or preventing or alleviating fatigue and/or increasing or improving nitric oxide signal or level in a subject.

22. The composition of claim 21, **characterized in that** the enhancing or improving muscle function comprises one or more of the following: alleviating or preventing muscle dysfunction, improving mitochondrial function in muscle, improving insulin resistance in muscle, increasing muscle mass, increasing muscle strength and endurance, improving glucose transport and metabolism in skeletal muscle, alleviating or preventing muscle injury, and reducing muscle fatigue; the improving exercise performance comprises one or more of the following: increasing running and/or swimming distance, increasing exercise time, increasing running and/or swimming speed, improving exercise endurance or explosive force, reducing inflammation and muscle fatigue after exercise, and improving muscle contraction during exercise; the preventing or alleviating fatigue comprises preventing physical fatigue after exercise and accelerating recovery after exercise.

23. The composition of claim 21 or 22, **characterized in that** the composition increases the level of nitrite and/or cyclic guanosine monophosphate.

24. The composition of claim 21 or 22, **characterized in that** the composition enhances or improves muscle function and/or improves exercise performance and/or prevents or alleviates fatigue by improving nitric oxide level or signal, or increasing the level of cyclic guanosine monophosphate, or increasing the level of nitrite.

25. The composition of any one of claims 21-24, **characterized in that** compared with a composition not comprising acetyltaurine or a hydrate, salt, ester, solvate, derivative thereof; or diarginine or a salt, ester, derivative thereof; or a mixture of both, the enhancement or improvement in muscle function or exercise performance and/or the prevention or alleviation of fatigue in a subject administered with the composition comprising acetyltaurine or a hydrate, salt, ester, solvate, derivative thereof; or diarginine or a salt, ester, derivative thereof; or a mixture of both is more than 10%, 30%, 40%, 50%, 60%, 70% or 90%.

26. The composition of any one of claims 21-24, **characterized in that** compared with a control subject not receiving administration, nitric oxide signal or level in a subject of administration group increases or improves more than 10%, 30%, 40%, 50%, 60%, 70%, 90%, 120%, 150% or 180%.

27. The composition of any one of claims 21-26, **characterized in that** in the composition, the amount of the acetyltaurine or hydrate, salt, ester, solvate, derivative thereof is 0.1 µM to 1 M or 0.05-20 g or 0.001 to 50.0 wt% (w/w); the amount of the diarginine or salt, ester, derivative thereof is 0.1 µM to 1 M or 0.1-30 g or 0.001 to 90.0 wt% (w/w).

28. The composition of any one of claims 21-27, **characterized in that** the composition further comprises a component for improving nitric oxide signal or level, which is coenzyme Q10, vitamin E, vitamin C, carnosine, lycopene, creatine antioxidant, arginine, cysteine, resveratrol, taurine, ergothioneine, glutathione.

29. A method for increasing or improving the level of nitrite and/or cyclic guanosine monophosphate, **characterized in that** the method comprises: administering acetyltaurine or a hydrate, salt, ester, solvate, derivative thereof; or diarginine or a salt, ester, derivative thereof; or a mixture of both to a subject.

30. The method of claim 29, **characterized in that** compared with a control subject not receiving administration, the level of nitrite and/or cyclic guanosine monophosphate in a subject of administration group increases or improves more than 10%, 30%, 40%, 50%, 60%, 70% or 90%.

31. The method of claim 29 or 30, **characterized in that** the subject is human or mammal.

32. The method of any one of claims 29-31, **characterized in that** the acetyltaurine or hydrate, salt, ester, solvate, derivative thereof; or the diarginine or salt, ester, derivative thereof; or the mixture of both is formulated as nutritional product, dietary supplement, food or beverage.

33. The method of any one of claims 29-32, **characterized in that** the acetyltaurine or hydrate, salt, ester, solvate, derivative thereof; or the diarginine or salt, ester, derivative thereof; or the mixture of both, is prepared into a solid preparation or a liquid preparation.

34. The method of any one of claims 29-33, **characterized in that** the acetyltaurine or hydrate, salt, ester, solvate, derivative thereof is administered at a dosage of 0.1 µM to 1 M or 0.05-20 g or 0.001 to 90.0 wt% (w/w); the diarginine or salt, ester, derivative thereof is administered at a dosage of 0.1 µM to 1 M or 0.1-30 g or 0.001 to 90.0 wt% (w/w).

35. Use of acetyltaurine or a hydrate, salt, ester, solvate, derivative thereof; or diarginine or a salt, ester, derivative thereof; or a mixture of both in a manufacture of a composition for increasing or improving the level of nitrite and/or cyclic guanosine monophosphate.

36. The use of claim 35, **characterized in that** compared with a control subject not receiving administration, nitric oxide signal or level in a subject of administration group increases or improves more than 10%, 30%, 40%, 50%, 60%, 70%, 90%, 120%, 150% or 180%.

37. The use of claim 35 or 36, **characterized in that** the acetyltaurine or hydrate, salt, ester, solvate, derivative thereof; or the diarginine or salt, ester, derivative thereof; or the mixture of both, is prepared into a solid preparation or a liquid preparation.
